# EUROPÄISCHE PATENTSCHRIFT

(11) **EP 0 691 326 B1**
(45) Veröffentlichungstag und Bekanntmachung des Hinweises auf die Patenterteilung: **14.10.1998**
(21) Anmeldenummer: 95109939.9
(22) Anmeldetag: 26.06.1995
(51) Int. Cl.: C07C 68/02, C07C 69/96

(54) **Verfahren zur Herstellung von Arylcarbonaten**
Process for the preparation of aryl carbonates
Procédé de préparation de carbonates d'aryle

(30) Priorität: 07.07.1994 DE 4423863
(43) Veröffentlichungstag der Anmeldung: 10.01.1996
(73) Patentinhaber: BAYER AG, 51368 Leverkusen (DE)
(72) Erfinder: Ooms, Pieter, Dr., D-47800 Krefeld (DE); Buysch, Hans-Josef, Dr., D-47809 Krefeld (DE); Schön, Norbert, Dr., D-64289 Darmstadt (DE)

(56) Entgegenhaltungen:
- WO-A-91/06526
- US-A- 5 239 105

## Beschreibung

Die Erfindung betrifft ein Verfahren zur Herstellung von Carbonaten mit aromatischen Estergruppen durch Umsetzung von aromatischen Monohydroxyverbindungen mit Phosgen oder Chlorkohlensäureestern aromatischer Monohydroxyverbindungen unter Abspaltung von Chlorwasserstoff in Gegenwart von Verbindungen der allgemeinen Formel AₓB_{y}O_{z} als heterogene Katalysatoren.

Carbonate mit aromatischen Estergruppen eignen sich zur Herstellung von Polycarbonaten nach dem Schmelzumestetungsverfahren, zur Herstellung von Phenylurethanen oder sind Vorprodukte von Wirkstoffen aus dem Pharma- und Pflanzenschutzsektor.

Es ist bekannt, daß Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann. In jedem Falle werden große Mengen Kochsalz als Nebenprodukt erhalten. Ferner muß Sorge für die Lösungsmittel-Rückgewinnung getragen werden.

Man hat daher eine Kondensation ohne Mitverwendung von Lösungsmitteln in Gegenwart von Tetramethylammoniumhalogeniden als Katalysatoren vorgeschlagen (US-A-2 837 555). Dabei sind daher die benötigten Katalysatormengen relativ groß. Man muß in der Regel mit 5 bis 7 Gew.-% Katalysator, bezogen auf die eingesetzte Phenolmenge, arbeiten, um wirtschaftliche Reaktionsgeschwindigkeiten zu erhalten; die Reaktionstemperaturen von 180°C bis 215°C bringen die Gefahr einer Zersetzung der thermolabilen Tetramethylammoniumhalogenide mit sich. Der Katalysator muß ferner anschließend durch Waschen mit Wasser entfernt werden, wodurch seine Rückgewinnung erheblich erschwert wird. Darüberhinaus wird weit mehr als die stöchiometrisch notwendige Menge an Phosgen verbraucht.

Nach einem weiteren Verfahren (US-A-3 234 263) werden Diarylcarbonate durch Erhitzen von Phenylchlorkohlensäureestern in Gegenwart großer Mengen (Erd)alkaliverbindungen mit tertiären Stickstoffbasen als Katalysatoren erhalten. Dieses Verfahren hat jedoch den Nachteil, daß man hohe Temperaturen anwenden und die Katalysatoren wie (Erd)Alkaliverbindungen teilweise lösen muß, um auch nur annähernd auf wirtschaftlich vertretbare Reaktionszeiten zu kommen. Bei diesem Verfahren geht die Hälfte des ursprünglich eingesetzten Phosgens in Form von CO₂ verloren. Außerdem muß man in einem vorgelagerten separaten Verfahrensschritt die Chlorkohlensäureester synthetisieren.

Nach der US-A-2 362 865 erhält man Diarylcarbonate durch Phosgenierung von aromatischen Hydroxyverbindungen in Gegenwart von metallischem Titan, Eisen, Zink und Zinn oder in Form ihrer löslichen Salze, besonders der Chloride und Phenolate. Obwohl sehr gute Ausbeuten erhalten werden, ist es schwierig, die Katalysatoren von den Produkten zu trennen. Man muß sogar bei Destillationen mit einer gewissen Flüchtigkeit dieser Verbindungen und auch mit thermischen Zersetzungen durch diese Verbindungen rechnen, die zu Verunreinigungen, Qualitätsminderungen und Ausbeuteeinbußen führen.

Somit erscheint es sinnvoll, heterogene, nicht lösliche Katalysatoren zu verwenden, die eine Aufarbeitung des Reaktionsgemisches wesentlich erleichtern. Auch dazu wurden Vorschläge gemacht. So wird nach der Lehre der EP-A-516 355 vor allem Aluminiumtrifluorid empfohlen, das gegebenenfalls auf Träger wie Alumosilikate aufgebracht wird. Die Synthese von Aluminiumfluorid ist jedoch durch die Handhabung von Fluor oder Flußsäure sehr aufwendig und teurer. Weiter werden in WO 91/06526 Metallsalze auf porösen Trägern als Katalysatoren für die erfindungsgemäße Umsetzungen beschrieben. Wie aus den Versuchsbeispielen hervorgeht, ist eine vollkontinuierliche Phosgenierung von Phenol an solchen Katalysatoren nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

In US-A 5,239,105 werden als heterogene Katalysatoren zur Herstellung von Diphenylcarbonat (DPC) Oxide des Wolframs oder der Metalle der Gruppe Vb des Periodensystems, Silicate der Gruppen IIa, IIIb, IVb, Vb, der Lanthaniden oder Actiniden oder Tonmineralien wie Montmorillonit vorgeschlagen. Die besten Ausbeuten an DPC werden bei Verwendung silicatischer Katalysatoren erhalten.

Es wurde nun gefunden, daß Verbindungen der allgemeinen Formel (III)

AₓB_{y}O_{z} (III),

in welcher
- A: für ein mono-, di- und/oder trivalentes Metallkation steht und
- B: für ein tri-, tetra-, penta- und/oder hexavalentes Metallkation steht und
- x: für eine Zahl von 1 bis 4 steht und
- y: für eine Zahl von 1 oder 2 steht und
- z: für eine Zahl von 3, 6, 7 oder 9 steht,
(im folgenden Metallate genannt), ausgezeichnete Katalysatoren für die Umsetzung von Phosgen oder Chlorkohlensäureestern mit aromatischen Hydroxyverbindungen darstellen. Dies ist besonders überraschend und unerwartet, weil nach der vorgängigen Lehre der WO 91/06526 Oxide von Metallen wie Titan und Zirkon bevorzugt als widerstandsfähige und inerte Trägermaterialien genannt werden.

Der Gegenstand der vorliegenden Erfindung ist demnach ein Verfahren zur Herstellung von Arylcarbonaten durch Umsetzung von aromatischen Monohydroxyverbindungen mit Phosgen oder Chlorkohlensäureestern aromatischer Monohydroxyverbindungen, dadurch gekennzeichnet, daß man die Umsetzung bei einer Temperatur im Bereich von 50 bis 450 °C, bei einem Druck von 0,05 bis 20 bar in Gegenwart einer oder mehrerer Verbindungen der allgemeinen Formel (III)

AₓB_{y}O_{z} (III),

in welcher
- A: für ein mono-, di- und/oder trivalentes Metallkation steht und
- B: für ein tri-, tetra-, penta- und/oder hexavalentes Metallkation steht und
- x: für eine Zahl von 1 bis 4 steht und
- y: für eine Zahl von 1 oder 2 steht und
- z: für eine Zahl von 3, 6, 7 oder 9 steht,
als heterogenen Katalysatoren durchführt.

Das erfindungsgemäße Verfahren hat den großen Vorteil, daß man den Katalysator sehr leicht abtrennen kann und keine Verunreinigungen im rohen Reaktionsprodukt verbleiben. Die Aufarbeitung wird dadurch wesentlich vereinfacht.

Aromatische Monohydroxyverbindungen für das erfindungsgemäße Verfahren sind solche der Formel

Ar¹-OH (I),

worin
- Ar¹: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch 1 oder 2 Substituenten wie geradkettiges oder verzweigtes C₁-C₄-Alkyl, geradkettiges oder verzweigtes C₁-C₄-Alkoxy, die mit Phenyl, Cyano und Halogen (z.B. F, Cl, Br) substituiert sein können und wobei weiterhin die heterocyclischen Reste mit einem ankondensierten Benzolkern verbunden sein können.

Beispiele für aromatische Monohydroxyverbindungen der Formel (I) sind: Phenol, o-, m- und p-Kresol, o-, m- und p-Isopropylphenol, die entsprechenden Halogen-bzw. Alkoxyphenole, wie p-Chlorphenol bzw. p-Methoxyphenol, ferner Monohydroxyverbindungen des Naphthalins, Anthracens und Phenanthrens, weiterhin 4-Hydroxy-pyridin und Hydroxychinoline. Vorzugsweise werden gegebenenfalls substituierte Phenole, ganz besonders bevorzugt Phenol selbst eingesetzt.

Das erfindungsgemäße Verfahren kann sowohl mit Phosgen als auch mit Chlorkohlensäureestern aromatischer Monohydroxyverbindungen durchgeführt werden. Für den Fall der Durchführung mit Phosgen entsteht zunächst der Chlorkohlensäureester der mit weiterer im Reaktionsgemisch vorhandener aromatischer Monohydroxyverbindung zum Diarylcarbonat umgesetzt wird.

Geht man von Chlorkohlensäureestern und einer aromatischen Monohydroxyverbindung aus, können symmetrische oder unsymmetrische Carbonate erhalten werden.

Geeignete aromatische Chlorkohlensäureester für das erfindungsgemäße Verfahren sind solche der Formel (II)

Ar¹-OCOCl (II),

worin Ar¹ die bei Formel (I) angegebene Bedeutung hat. Metallate im Sinne der Erfindung sind Verbindungen der allgemeinen Formel (III)

AₓB_{y}O_{z} (III),

in welcher
- A: für ein mono-, di- und/oder trivalentes Metallkation steht und
- B: für ein tri-, tetra-, penta- und/oder hexavalentes Metallkation steht und
- x: für eine Zahl von 1 bis 4 steht und
- y: für eine Zahl von 1 oder 2 steht und
- z: für eine Zahl von 3, 6, 7 oder 9 steht.

Als Beispiele für Metallkation A seien genannt: monovalente Metallkationen wie Li, Na, K, Rb, Cs, Cu, Ag und Tl, divalente Metallkationen wie Be, Mg, Ca, Sr, Ba, Zn, Hg, Sn, Pb, Fe, Mn, Co, Ni, trivalente Metallkationen wie B, Al, Ga, Y, Tl, Bi, Fe, Mn, Co, Cr, V, Mo, bevorzugt sind Na, K, Be, Mg, Ca, Ba, Fe, Ni, Zn, Al, Pb, Cr, V und Mo, besonders bevorzugt sind Na, K, Mg, Ca, Ba, Fe, Pb, Ni und Zn.

Als Beispiele für Metallkation B seien genannt: trivalente Metallkationen wie Sc, Y, Cr, Mn, Fe und Ga, tetravalente Metallkationen wie Ti, Zr, Hf, V, Nb, Mo, Ru, Os, Re, Ir, Sn und Pb, pentavalente Metallkationen wie V, Nb, Ta, Re, Ru, Os, Rh, Ir, Sb und Bi, hexavalente Metallkationen wie Mo, W und Te, bevorzugt sind Ti, Zr, Nb, Ta, V, Mo, W und Sn, besonders bevorzugt sind Ti, Zr, Nb und Ta.

In den Metallaten können eine oder mehrere Metallkationen A oder Metallkationen B auch in verschiedener Wertigkeit nebeneinander vorkommen.

Besonders geeignet als heterogene Katalysatoren sind Metallate mit Perowskit- und Pyrochlorstruktur.

Sie können in kristalliner Form in verschiedenen Modifikationen vorliegen. Sie können ganz oder teilweise amorph sein.

Solche Metallate und deren Herkunft bzw. Herstellverfahren für solche Verbindungen sind beispielsweise in Kirk-Othmer, Encyclopedia of Chemical Technology, 2. Auflage, Bd. 20, S. 410 ff, Bd. 22, S. 656, Supplement Vol., S. 153, Bd. 19, S. 651 ff, Bd. 13, S. 768, 782 ff., New York 1967/1971, Ullmann's Encyclopedia of industrial chemistry, 3. Aufl.., Bd. 17, S. 431, Weinheim 1966 und EP 0 547 791, 0 575 745 beschrieben.

Dabei kommen sowohl Metallate aus natürlichen Quellen, d.h. verschiedene Mineralien wie z.B. Ilmenit oder Perowskit aber auch synthetische wie solche aus Vorprodukten, beispielsweise Metallsalzen, Metalloxiden und Metallalkoxiden in Frage.

Bei den im erfindungsgemäßen Verfahren einzusetzenden Metallaten handelt es sich bevorzugt um Titanate, Zirkonate, Niobate und Tantalate, beispielsweise Lithiumtitanat, Natriumtitanat, Kaliumtitanat, Bariumtitanat, Strontiumtitanat, Calciumtitanat (vorkommend als Perovskit in monokliner, pseudokubischen Form), Magnesiumtitanat (vorkommend in rhomboedrischer Form), Aluminiumtitanat (vorkommend als Tielit in sowohl α- als auch β-Modifikation), Cadmiumtitanat (vorkommend in orthorhombischer und kubischer Struktur), Cobalttitanat (vorkommend in rhomboedrischer und kubischer Form), Eisentitanat (vorkommend als Ilmenit oder Pseudobrookit), Mangantitanat (vorkommend in hexagonaler und kubischer Form), Nickeltitanat, Zinktitanat (Spinell-Form), Bleititanat (rhombische Pyramide), Lithiumzirkonat, Natriumzirkonat, Kaliumzirkonat, Bariumzirkonat, Calciumzirkonat, Strontiumzirkonat, Eisenzirkonat, Lanthanzirkonat, Ytriumzirkonat, Natriumniobat, Kaliumniobat, Calciumniobat, Magnesiumniobat, Zinkniobat, Eisenniobat (vorkommend als Columbit), Nickelniobat, Bleiniobat, Natriumtantalat, Kaliumtantalat, Bleitantalat, Magnesiumstannat, Calciumwolframat, Bariumwolframat, Zinkmolybdat, Bleimolybdat, auch gemischte Metallate wie Bleimagnesiumniobat, Eisenmanganniobat, Bleimagnesiumtantalat und Bariumzinktantalat.

Die Metallate im Sinne der Erfindung können getrocknet, teilgetrocknet oder als Hydrate eingesetzt werden.

Durch sukzessive Entwässerung (Calcinierung) von gemischten Metallhydroxiden und Metalloxidhydroxiden bei Temperaturen von 80 bis oberhalb von 1200°C entstehen zunächst teilentwässerte Metallate, die noch merkliche Mengen an Hydroxylgruppen enthalten und mit fortschreitender Entwässerung in die wasserfreien Metallate übergehen. Je nach Art des Ausgangshydroxids, bzw. -oxidhydroxids können bei der Calcinierung verschiedene der obengenannten Modifikationen des Metallats durchlaufen werden.

Bevorzugte Metallate besitzen BET-Oberflächen von 0,1 bis 500 m²/g, besonders bevorzugt solche von 0,5 bis 450 m²/g und ganz besonders bevorzugte solche von 1 bis 400 m²/g. Es können saure, neutrale und basische Metallate verwendet werden.

Die Katalysatoren können z.B. als Pulver oder Formkörper eingesetzt werden und nach der Umsetzung z.B. durch Filtration, Sedimentation oder Zentrifugieren abgetrennt werden. Für den Fall der Anordnung als Festbett werden die Metallate vorzugsweise als Formkörper, z.B. als Kugeln, Zylinder, Stäbchen, Hohlzylinder, Ringe usw. eingesetzt.

Die Metallat-Katalysatoren werden beim Arbeiten mit suspendiertem Katalysator in Rührgefäßen oder Blasensäulen in Mengen von 0,5 bis 100 Gew.-%, bevorzugt von 5 bis 100 Gew.-% und besonders bevorzugt von 5 bis 50 Gew.-%, bezogen auf die eingesetzte Menge an Monohydroxyverbindung, verwendet.

Im Falle einer kontinuierlichen Fahrweise im Gegen- oder Gleichstrom oder in der Rieselphase oder in der Gasphase am Festbettkatalysator werden Katalysatorbelastungen von 0,1 bis 20 g aromatische Hydroxyverbindung pro g Katalysator pro Stunde, bevorzugt 0,2 bis 10 g · g⁻¹ · h⁻¹ und besonders bevorzugt von 0,2 bis 5 g · g⁻¹ · h⁻¹ verwendet.

Die in diskontinuierlichen Versuchen verwendeten Metallate können bei gleichen Einsatzstoffen ohne Reinigung wiederholt eingesetzt werden. Bei einem Wechsel der Einsatzstoffe werden die Metallate zweckmäßig durch Extrahieren mit inerten Lösungsmitteln, wie sie beispielsweise weiter unten als Reaktionsmedien genannt sind, oder mit Alkoholen, wie Methanol, Ethanol, Isopropanol oder Butanol, mit Estern oder Amiden der Essigsäure oder durch Behandlung mit überhitztem Wasserdampf oder Luft gereinigt.

Bei kontinuierlicher Arbeitsweise können die eingesetzten Metallate über lange Zeit im Reaktor verbleiben. Eine Regenerierung kann gegebenenfalls durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 20 Gew.-% Sauerstoff enthaltenden Verdünnungsgasen wie Stickstoff, oder Kohlendioxid oder durch Kohlendioxid allein bei 200 bis 800°C erfolgen. Die bevorzugte Regenerierungstemperatur liegt bei 150 bis 700°C, besonders bevorzugt bei 200 bis 600°C.

Das erfindungsgemäße Verfahren wird bei einer Temperatur im Bereich von 50 bis 450°C, bevorzugt 100 bis 400°C, besonders bevorzugt 100 bis 350°C durchgeführt. Während der Durchführung des erfindungsgemäßen Verfahrens kann die Temperatur im genannten Bereich verändert, in bevorzugter Weise erhöht werden.

Das erfindungsgemäße Verfahren wird bei einem Druck von 0,05 bis 20 bar, vorzugsweise 1 bis 5 bar, durchgeführt.

Man kann das erfindungsgemäße Verfahren unter Mitwirkung von Lösungsmitteln wie aliphatischen und aromatischen Kohlenwasserstoffen, wie Pentan, Hexan, Octan, Benzol, isomeren Xylolen, Diethylbenzol, Alkylnaphthalinen, Biphenyl; halogenierten Kohlenwasserstoffen, wie Dichlormethan, Trichlorethylen usw. durchführen.

Man kann das erfindungsgemäße Verfahren sowohl in der Gasphase als auch in der Flüssigphase durchführen.

Vorzugsweise wird das Verfahren in der Schmelze durchgeführt, beispielsweise, indem man in eine Suspension eines Metallats in einer Schmelze der aromatischen Monohydroxyverbindung der Formel (I) Phosgen oder einen Chlorkohlensäureester der Formel (II) einleitet und nach Beendigung der Reaktion den Katalysator z.B. durch Filtration oder Zentrifugieren abtrennt.

Das Verfahren wird in der Gasphase durchgeführt, indem man Phosgen und Phenol verdampft und das Gemisch über ein Bett eines in einem Rohr angeordneten stückigen Katalysators leitet.

Eine weitere bevorzugte Ausführungsform der Synthese ist die Begasung einer Schmelze der aromatischen Monohydroxyverbindung der Formel (I) mit darin suspendiertem Metallat-Katalysator mit Phosgen oder Phosgen-Chlorwasserstoff-Gemischen oder mit Chlorkohlensäureestern der Formel (II) in einer kontinuierlich arbeitenden Blasensäule bzw. Blasensäulen-Kaskade.

Eine weitere bevorzugte Durchführungsform ist das Gleichstromverfahren, bei dem aromatische Hydroxyverbindungen der Formel (I) und Phosgen bzw. Chlorkohlensäureester der Formel (II) im Gleichstrom beispielsweise von oben her auf eine in einem Rohr angeordnete Katalysatorschüttung aufgebracht und unten am Fuß des Rohres Chlorwasserstoff und Phosgenierungsprodukte abgezogen werden.

Eine weitere bevorzugte Ausführungsform mit besonders günstigen Ergebnissen ist die Durchführung der erfindungsgemäßen Umsetzung im Gegenstrom in der Rieselphase, wobei die aromatische Monohydroxyverbindung der Formel (I) als Schmelze oder in Form einer Lösung oben auf ein Bett von Metallat gegeben wird und diesem Flüssigkeitstrom von unten ein Strom von Phosgen oder Chlorkohlensäureester entgegengeschickt wird. Zweckmäßig wird diese Ausführungsform in einem senkrecht stehenden Rohrreaktor durchgeführt, die auch Zwischenböden zur verbesserten Verteilung von Gas- und Flüssigkeitsstrom enthalten kann.

Eine weitere bevorzugte Ausführungsform ist das Gasphasenverfahren bei Temperaturen von 150 bis 450°C, bevorzugt 200 bis 350°C mit Drucken von 0,05 bis 20, bevorzugt 0,1 bis 4 bar, besonders bevorzugt 0,1 bis 3 bar.

Bei diesem Verfahren wird der Druck so mit der Temperatur variiert, daß die Komponenten in der Gasphase bleiben und nicht auf der Katalysatorschüttung kondensieren.

Das molare Verhältnis der Reaktionspartner aromatische Monohydroxyverbindungen der Formel (I) zu Phosgen beträgt 0,5 bis 8:1, bevorzugt 1,5 bis 3:1. Das äquivalente molare Verhältnis ist in diesem Fall 2:1.

In entsprechender Weise wird die aromatische Monohydroxyverbindung mit einem Chlorkohlensäureester im Molverhältnis von 0,25 bis 4:1, bevorzugt 0,8 bis 1,5:1 umgesetzt. In diesem Fall beträgt das molare Verhältnis 1:1.

Das durch heterogene Katalyse gewonnene rohe aromatische Carbonat ist häufig schon sehr rein und kann nach Entgasung von restlichem Chlorwasserstoff oder anderen flüchtigen Stoffen bereits in dieser Form für viele Zwecke verwendet werden. Für anspruchsvollere Anwendungen kann das Carbonat gegebenenfalls z.B. durch Destillation oder Kristallisation weiter gereinigt werden.

### Beispiele

### Beispiel 1

In einem Planschlifftopf mit Strombrechern, einem Begasungsrührer und Rückflußkühler wurden 141 g (1,50 mol) Phenol in Gegenwart von 14,1 g (10 Gew.-% bezogen auf Phenol) eines pulverförmigen Magnesiumtitanats der Firma Bayer bei 140°C mit 0,75 mol/h Phosgen kontinuierlich begast. Nach etwa 2 h Reaktionszeit betrug der Phenolumsatz 36,4 %, wobei nur (58,3 g) Diphenylcarbonat gebildet wurde. Die Selektivität betrug >99 %.

### Beispiel 2

Das Beispiel 1 wurde mit 14,1 g eines pulverförmigem Bariumtitanats der Firma Aldrich bei 140°C wiederholt. Nach 2 h Reaktionszeit betrug der Phenolumsatz 31,6 %, wobei nur (50,1 g) Diphenylcarbonat gebildet wurde. Die Selektivität betrug ca. 99 %.

### Beispiel 3

Das Beispiel 1 wurde mit 14,1 g eines pulverförmigen Bleititanats der Firma Aldrich bei 140°C wiederholt. Nach 2 h Reaktionszeit betrug der Phenolumsatz 39,3 %, wobei nur (62,8 g) Diphenylcarbonat gebildet wurde. Die Selektivität war größer als 99 %.

### Beispiel 4

Das Beispiel 1 wurde mit 14,1 g eines pulverförmigen Natriumtitanats der Firma Bayer bei 140°C wiederholt. Nach 2 h Reaktionszeit betrug der Phenolumsatz 21,5 %, wobei nur (34,1 g) Diphenylcarbonat gebildet wurden. Die Selektivität war ca. 99 %.

### Beispiel 5

Das Beispiel 1 wurde mit 14,1 g eines pulverförmigen Calciumtitanats der Firma Aldrich bei 140°C wiederholt. Nach 2 h Reaktionszeit betrug der Phenolumsatz 35,4 %, wobei 51,6 g Diphenylcarbonat gebildet wurden. Die Selektivität war ca. 91 %.

### Beispiel 6

Das Beispiel 1 wurde mit 14,1 g eines pulverförmigen Magnesiumniobates der Firma Starck bei 140°C wiederholt. Nach 2 h Reaktionszeit betrug der Phenolumsatz 16,0 %, wobei 2,1 g Chlorameisensäurephenylester und 24,1 g Diphenylcarbonat gebildet wurden. Die Selektivität zu Kohlensäureestern war größer als 99 %.

### Beispiel 7

Das Beispiel 1 wurde mit 14,1 g eines pulverförmigen Zinkniobats der Firma Starck bei 140°C wiederholt. Nach 2 h Reaktionszeit betrug der Phenolumsatz 6,3 %, wobei 1,1 g Chlorameisensäurephenylester und 9,2 g Diphenylcarbonat gebildet wurden. Die Selektivität zu Kohlensäureestern war ca. 98 %.

### Beispiel 8

Das Beispiel 1 wurde mit 14,1 g eines pulverförmigen Eisenniobats der Firma Starck bei 140°C wiederholt. Nach 2 h Reaktionszeit betrug der Phenolumsatz 4,1 %, wobei 2,3 g Chlorameisensäurephenylester und 4,2 g Diphenylcarbonat gebildet wurde. Die Selektivität zu Kohlensäureestern war ca. 90 %.

### Beispiel 9

Das Beispiel 1 wurde mit 14,1 g eines pulverförmigen Bariumzinktantalats der Firma Starck bei 140°C wiederholt. Nach 2 h Reaktionszeit betrug der Phenolumsatz 8,4 %, wobei 3,4 g Chlorameisensäurephenylester und 10,8 g Diphenylcarbonat gebildet wurden. Die Selektivität zu Kohlensäureestern war ca. 97 %.

### Beispiel 10 (zum Vergleich)

Das Beispiel 1 wurde ohne Zusatz von Metallat bei 140°C wiederholt. Nach 2 h Reaktionszeit betrug der Phenolumsatz weniger als 0,2 %.

### Beispiel 11

In einem Dreihalskolben mit Thermometer und Rückflußkühler erhitzt man ein Gemisch aus 9,4 g (0,10 mol) Phenol und 15,7 g (0,10 mol) Chlorameisensäurephenylester in Gegenwart von 0,94 g (10 Gew.-% bezogen auf Phenol) eines pulverförmigen Magnesiumtitanats der Fa. Bayer auf 100°C. Nach 5 h Reaktionszeit wird ein Phenolumsatz von 78,1 % zu Diphenylcarbonat gefunden. Die Carbonatselektivität war >99 %.

### Beispiel 12

Das Beispiel 11 wurde mit demselben Katalysator bei 120°C wiederholt. Nach 2 h Reaktionszeit betrug der Phenolumsatz zu Diphenylcarbonat 88,3 %. Die Carbonatselektivität war >99 %.

### Beispiel 13

Das Beispiel 11 wurde mit demselben Katalysator bei 140°C wiederholt. Nach 0,5 h Reaktionszeit betrug der Phenolumsatz zu Diphenylcarbonat 45,5 %. Die Carbonatselektivität war >99 %.

### Beispiel 14

Das Beispiel 11 wurde mit demselben Katalysator bei 160°C wiederholt. Nach 1 h Reaktionszeit betrug der Phenolumsatz zu Diphenylcarbonat 97,8 %. Die Carbonatselektivität war >99 %.

### Beispiel 15

Das Beispiel 11 wurde mit 0,94 g eines pulverförmigen Bariumtitanats der Fa. Aldrich bei 160°C wiederholt. Nach 0,5 h Reaktionszeit betrug der Phenolumsatz zu Diphenylcarbonat 88,2 %. Die Carbonatselektivität war >99 %.

### Beispiel 16

Das Beispiel 11 wurde mit 0,94 g eines Bleititanats der Fa. Aldrich bei 160°C wiederholt. Nach 0,5 h Reaktionszeit betrug der Phenolumsatz zu Diphenylcarbonat 99,2 %. Die Carbonatselektivität war >99 %.

### Beispiel 17

Das Beispiel 11 wurde mit 0,94 g eines pulverförmigen Natriumtitanats der Fa. Bayer bei 160°C wiederholt. Nach 0,5 h Reaktionszeit betrug der Phenolumsatz zu Diphenylcarbonat 82,2 %. Die Carbonatselektivität war >99 %.

### Beispiel 18

Das Beispiel 11 wurde mit 0,94 g eines pulverförmigen Calciumtitanats der Fa. Aldrich bei 140°C wiederholt. Nach 0,5 h Reaktionszeit betrug der Phenolumsatz zu Diphenylcarbonat 89,1 %. Die Carbonatselektivität war >99 %.

### Beispiel 19

Das Beispiel 11 wurde mit 0,94 g eines pulverförmigen Magnesiumniobats der Fa. Starck bei 160°C wiederholt. Nach 3 h Reaktionszeit betrug der Phenolumsatz zu Diphenylcarbonat 64,7 %. Die Carbonatselektivität war >99 %.

### Beispiel 20

Das Beispiel 11 wurde mit 0,94 g eines pulverförmigen Zinkniobats der Fa. Starck bei 160°C wiederholt. Nach 6 h Reaktionszeit betrug der Phenolumsatz zu Diphenylcarbonat 28,6 %. Die Carbonatselektivität war >99 %.

### Beispiel 21

Das Beispiel 11 wurde mit 0,94 g eines Eisenniobats der Fa. Starck bei 160°C wiederholt. Nach 5 h Reaktionszeit betrug der Phenolumsatz zu Diphenylcarbonat 65,5 %. Die Carbonatselektivität war ca. 97 %.

### Beispiel 22

Das Beispiel 11 wurde mit 0,94 g eines pulverförmigen Nickelniobats der Fa. Starck bei 160°C wiederholt. Nach 6 h Reaktionszeit betrug der Phenolumsatz zu Diphenylcarbonat 16,8 %. Die Carbonatselektivität war >99 %.

### Beispiel 23

Das Beispiel 11 wurde mit 0,94 g eines pulverförmigen Bariumzinktantalats der Fa. Starck bei 160°C wiederholt. Nach 4 h Reaktionszeit betrug der Phenolumsatz zu Diphenylcarbonat 74,9 %. Die Carbonatselektivität war ca. 98 %.

## Patentansprüche

1. Verfahren zur Herstellung von Arylcarbonaten durch Umsetzung von aromatischen Monohydroxyverbindungen mit Phosgen oder Chlorkohlensäureestern aromatischer Monohydroxyverbindungen, dadurch gekennzeichnet, daß man die Umsetzung bei einer Temperatur im Bereich von 50 bis 450 °C, bei einem Druck von 0,05 bis 20 bar in Gegenwart einer oder mehrerer Verbindungen der allgemeinen Formel
AₓB_{y}O_{z} (III),
A für ein mono-, di und/oder trivalentes Metallkation steht und
B für ein tri-, tetra-, penta- und/oder hexavalentes Metallkation steht und
x für eine Zahl von 1 bis 4 steht und
y für eine Zahl von 1 oder 2 steht und
z für eine Zahl von 3, 6, 7 oder 9 steht,
als heterogenen Katalysatoren durchführt.

2. Verfahren nach Anspruch 1, dadurch gekennzeichnet, daß die heterogenen Katalysatoren nach der BET-Methode bestimmte Oberflächen von 0,1 bis 500 m²/g aufweisen und in Mengen von 0,5 bis 100 Gew.-% bezogen auf die Menge an Monohydroxyverbindung, bei nicht vollkontinuierlicher Fahrweise bzw. mit Belastungen von 0,1 bis 20 g Monohydroxyverbindung pro g Katalysator pro Stunde bei vollkontinuierlicher Fahrweise, eingesetzt werden.

## Claims

1. Process for the production of aryl carbonates by reacting aromatic monohydroxy compounds with phosgene or chloroformic acid esters of aromatic monohydroxy compounds, characterised in that the reaction is performed at a temperature in the range from 50 to 450°C, at a pressure of 0.05 to 20 bar in the presence of one or more compounds of the general formula
AₓB_{y}O_{z} (III),
in which
A denotes a mono-, di- and/or trivalent metal cation and
B denotes a tri-, tetra-, penta- and/or hexavelent metal cation and
x denotes a number from 1 to 4 and
y denotes a number of 1 or 2 and
z denotes a number of 3, 6, 7 or 9,
as heterogeneous catalysts.

2. Process according to claim 1, characterised in that the heterogeneous catalysts have surface areas determined using the BET method of 0.1 to 500 m²/g and are used in quantities of 0.5 to 100 wt.%, relative to the quantity of monohydroxy compounds, in the event of incompletely continuous operation, or at loadings of 0.1 to 20 g of monohydroxy compound per g of catalyst per hour in the event of completely continuous operation.

## Revendications

1. Procédé pour la préparation d'arylcarbonates par mise en réaction de composés monohydroxylés aromatiques avec du phosgène ou avec des esters chlorocarboniques de composés monohydroxylés aromatiques, caractérisé en ce qu'on effectue la mise en réaction à une température dans le domaine de 50 à 450°C, sous une pression de 0,05 à 20 bar, en présence d'un ou de plusieurs composés répondant à la formule générale
AₓB_{y}O_{z} (III)
dans laquelle
A représente un cation métallique mono-, di-et/ou trivalent, et
B représente un cation métallique tri-, tétra-, penta- et/ou hexavalent, et
x représente un nombre de 1 à 4, et
y représente le nombre 1 ou 2, et
z représente un nombre égal à 3, 6, 7 ou 9,
à titre de catalyseurs hétérogènes.

2. Procédé selon la revendication 1, caractérisé en ce que les catalyseurs hétérogènes présentent, conformément au procédé BET, des surfaces définies de 0,1 à 500 m²/g et sont mis en oeuvre dans des quantités de 0,5 à 100% en poids rapportés à la quantité du composé monohydroxylé, dans un mode opératoire non complètement en continu, respectivement avec des charges de 0,1 à 20 g de composé monohydroxylé par gramme de catalyseur par heure dans un mode opératoire complètement en continu.
